# DEMANDE DE BREVET EUROPEEN

(11) **EP 1 166 729 A1**
(43) Date de publication de la demande: **02.01.2002**
(21) Numéro de dépôt: 01420145.3
(22) Date de dépôt: 28.06.2001
(51) Int. Cl.: A61F 13/495, A61F 13/494

(54) **Couche à usage unique**

(30) Priorité: 29.06.2000 FR 0008392
(71) Demandeur: Prod'hygia S.A., 59494 Petite Foret (FR)
(72) Inventeur: Lorthioit, Bruno, 59171 Hornaing (FR); Comte, Tony, 69220 St Jean D'Ardieres (FR)

(57) **Abrégé**

Couche (1) à usage unique, comportant une feuille de fond (2), un matelas absorbant (3) et une feuille de dessus (4) perméable aux liquides. Ladite couche comporte deux bandes intérieures (9) pourvues d'élastiques sur leur largeur, au moins dans la région d'entrejambe, un bord latéral de chaque bande (9) étant assujetti au matelas absorbant et l'autre bord latéral étant assujetti à un voile de liaison (5) solidaire de la feuille de fond, ladite feuille de dessus comportant des parties latérales (4b), rabattues sur elles-mêmes autour d'élastiques (8) pour constituer les bandes intérieures, et chaque voile de liaison comportant un bord distal libre (10), chaque bande intérieure étant fixée au voile de liaison correspondant, en retrait de son bord distal et sur sa face intérieure.

## Description

La présente invention concerne une couche à usage unique, du type comportant une feuille de fond, un matelas absorbant et une feuille de dessus perméable aux liquides.

L'invention a pour objet de proposer une couche de ce type, qui soit de construction simple, tout en étant confortable et performante.

La nouvelle couche selon l'invention se caractérise par le fait qu'elle comporte deux bandes inférieures pourvues d'élastiques sur leur largeur, au moins dans la région d'entrejambes, un bord latéral de chaque bande étant assujetti au matelas absorbant et l'autre bord latéral étant assujetti à un voile de liaison solidaire de la feuille de fond, la feuille supérieure comportant des parties latérales rabattues sur elles-mêmes autour d'élastiques pour constituer les bandes intérieures, chaque voile de liaison comportant un bord distal libre, chaque bande intérieure étant fixée au voile de liaison correspondant, en retrait de son bord distal et sur sa face intérieure.

Les bandes inférieures précitées forment, avec la région centrale de la feuille de dessus, des barrières entre lesquelles les matières solides peuvent s'accumuler, et la présence d'élastiques répartis sur leur largeur permet d'obtenir une bonne étanchéité sans exercer pour autant une pression de contact susceptible de gêner l'utilisateur.

On forme ainsi une lèvre d'étanchéité, améliorant encore la retenue des liquides et/ou des solides.

Dans une réalisation particulière, les bandes intérieures s'étendent au-dessus du matelas absorbant.

Toujours dans une réalisation particulière, chaque voile de liaison est assemblé avec la feuille de fond autour d'au moins un élastique, de manière à constituer une bande de jambe élastiquement susceptible de contraction.

De telles bandes de jambe constituent des barrières supplémentaires vis-à-vis des liquides et/ou des matières solides.

Dans une réalisation particulière, les bandes intérieures sont réalisées d'un seul tenant avec la feuille supérieure.

Ainsi, la fabrication de la couche est simplifiée.

En outre, une telle particularité de réalisation est avantageuse puisque les liquides peuvent traverser la portion de la feuille supérieure qui s'étend entre le matelas absorbant et les bandes intérieures, et atteindre les régions latérales du matelas absorbant qui s'étendent sous les bandes intérieures, pour y être absorbés.

De préférence, la feuille supérieure est fixée au matelas absorbant le long d'une région centrale de ce dernier, s'étendant entre les bandes intérieures précitées.

De plus, en utilisant pour constituer chaque voile de liaison un matériau imperméable ou hydrophobe, par exemple un non-tissé hydrophobe, on évite les risques de fuite par effet de mèche.

L'utilisation d'un non-tissé est également avantageuse puisqu'elle permet un passage d'air sous la couche, ce qui contribue à accroître encore le confort.

De préférence, la feuille supérieure est hydrophile, de manière à ne pas gêner la diffusion des liquides vers le matelas absorbant.

De préférence également, la feuille de fond est imperméable, de manière à limiter les risques de fuite à travers la couche au cas où tous les liquides ne seraient pas retenus par le matelas absorbant.

Les voiles de liaison peuvent être réalisés dans le même matériau que la feuille de fond.

Les voiles de liaison peuvent être rapportés ou formés par pliage de la feuille de fond.

L'invention sera mieux comprise à la lecture de la description détaillée qui va suivre, d'exemples non limitatifs, et à l'examen du dessin annexé, sur lequel :
- la figure 1 est une vue schématique et partielle, en perspective, d'une couche à usage unique conforme à un premier exemple de mise en oeuvre de l'invention,
- la figure 2 est une vue en section selon le trait de coupe II-II de la figure 1,
- la figure 3 illustre l'assemblage des différents éléments constitutifs de la couche représentée sur les figures 1 et 2,
- la figure 4 est une vue analogue à la figure 2, illustrant une variante de réalisation, et
- la figure 5 représente une variante de réalisation.

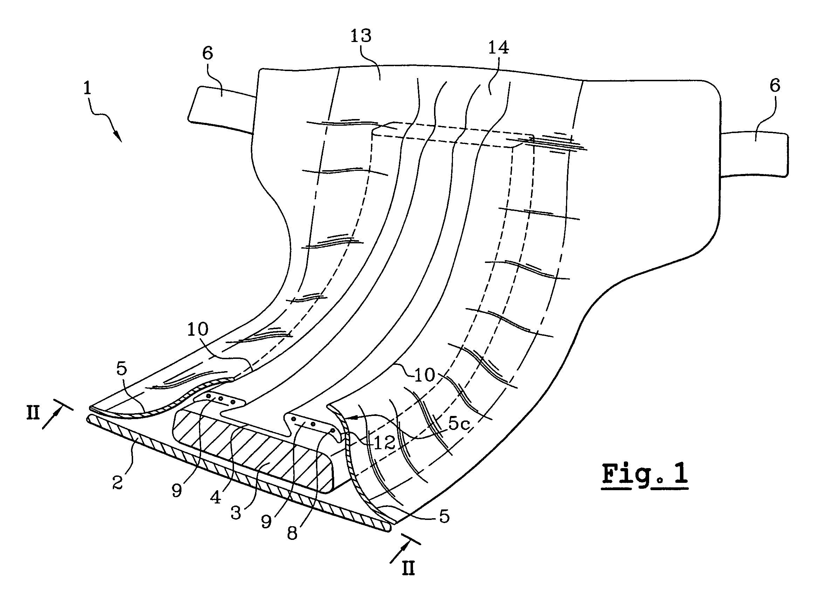

On a représenté sur la figure 1 la moitié d'une couche-culotte 1 à usage unique, conforme à l'invention, destinée à un enfant ou à un adulte incontinent.

Cette couche 1 comporte une feuille de fond 2 constituée dans l'exemple décrit par une feuille d'un matériau imperméable, par exemple un film de polyéthylène.

Cette feuille de fond 2 définit la face extérieure de la couche lorsque celle-ci est en place sur l'utilisateur.

La couche 1 comporte sur la feuille de fond 2 un matelas absorbant 3, composé de fibres naturelles ou synthétiques mixées avec des particules de superabsorbant, capable de retenir un volume important de liquides.

La couche 1 comporte également, recouvrant le matelas absorbant 3, une feuille supérieure 4 perméable aux liquides et de chaque côté de cette feuille supérieure 4 un voile de liaison 5 dont la fonction sera décrite plus loin.

La couche 1 comporte des moyens de fixation permettant de la maintenir en place sur l'utilisateur, ces moyens de fixation étant constitués par exemple, de manière conventionnelle, par des bandes de fixation 6 pourvues d'un adhésif, ou de moyens d'accrochage à crochets ou boucles.

Les moyens de fixation 6 sont agencés pour coopérer avec des moyens de réception adaptés présents sur la face extérieure de la partie de devant de la couche 1, c'est-à-dire la région qui se positionne sur le ventre de l'utilisateur.

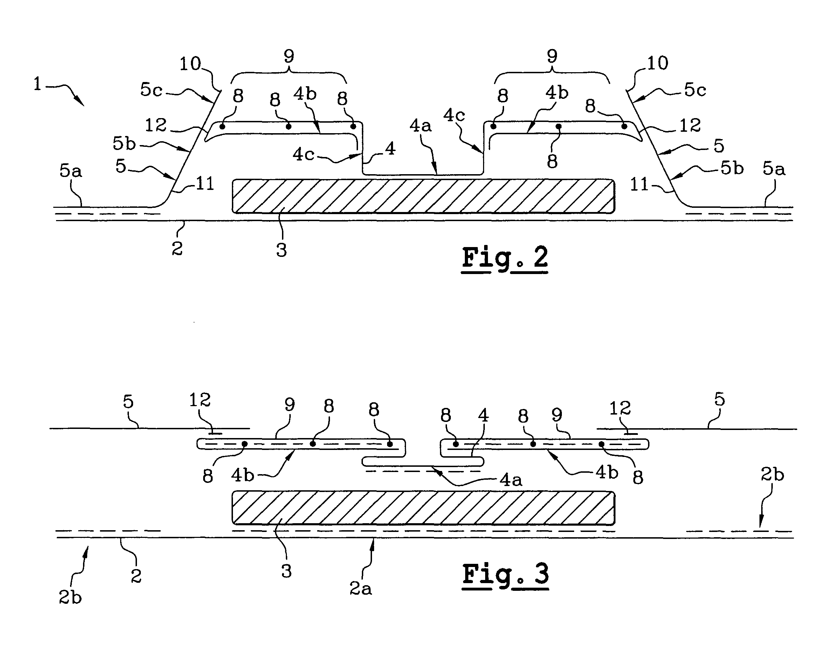

Si l'on se réfère maintenant à la figure 2, on remarque que la feuille supérieure 4 comporte une région centrale 4a fixée sur le matelas absorbant 3 et deux parties latérales 4b, rabattues sur elles-mêmes autour d'élastiques 8 pour constituer des bandes intérieures 9.

Chaque voile de liaison 5 comporte une portion proximale 5a fixée sur la feuille de fond 2 et une portion distale 5b dont le bord distal 10 est libre.

Chaque bande intérieure 9 est fixée sur le voile de liaison 5 correspondant le long d'une ligne de fixation 12, espacée d'une certaine distance du bord distal 10.

La portion 5c de chaque voile de liaison 5 qui s'étend entre le bord distal 10 et la ligne de fixation 12 constitue une lèvre d'étanchéité.

Dans l'exemple de réalisation décrit, les élastiques 8 sont répartis sur sensiblement toute la largeur de chaque bande intérieure 9.

Le nombre d'élastiques 8 utilisés est de préférence compris entre deux et cinq, étant égal à trois dans l'exemple illustré.

L'utilisation d'un nombre relativement élevé d'élastiques 8 permet de répartir la pression de contact sur la peau et contribue ainsi au confort de la couche.

De plus, l'utilisation de plusieurs élastiques 8 permet de renforcer l'étanchéité, en créant des barrières multiples s'opposant au déplacement des matières solides.

Lorsque la couche 1 est utilisée, les élastiques 8 sont contractés et tendent à éloigner les bandes intérieures 9 du matelas absorbant 3, de sorte que les portions 4c de la feuille supérieure 4 qui s'étendent entre le matelas absorbant 3 et les bandes intérieures 9 se tendent et forment des barrières qui s'opposent au déplacement de la matière solide vers les ouvertures des jambes.

Les liquides peuvent aisément traverser la feuille supérieure 4 pour atteindre le matelas absorbant 3.

Les portions 4c constituent des barrières vis-à-vis des matières solides mais peuvent être traversées par les liquides, lesquels sont alors absorbés par les parties latérales du matelas absorbant 3 qui s'étendent sous les bandes intérieures 9.

Les lèvres d'étanchéité 5c diminuent encore les risques de fuite de matières solides ou de liquides.

Pour assembler les différents éléments constitutifs de la couche 1, on procède comme suit.

Comme illustré sur la figure 3, on encolle la feuille de fond 2 sur sa face supérieure, dans sa région centrale 2a et le long de ses bords longitudinaux 2b, et l'on colle le matelas absorbant 3 sur la région centrale 2a.

Ensuite, on constitue les bandes intérieures 9 en déposant de la colle le long des parties latérales 2 de la feuille supérieure 4 puis en rabattant ces dernières sur elles-mêmes, après avoir positionné les élastiques 8.

Les bandes de non-tissé destinées à constituer les voiles de liaison 5 sont fixées par soudure ou collage sur les bandes intérieures 9 le long des lignes de fixation 12.

Ensuite, la région centrale 4a de la feuille supérieure 4 est encollée puis fixée sur le matelas absorbant 3.

Les voiles de liaison 5 sont fixés au niveau des extrémités longitudinales 13 de la couche sur la feuille de fond 2, comme illustré sur la figure 1.

Les extrémités longitudinales 14 des rabats de la feuille supérieure 4 sont fixées sur la feuille de fond 2, le matelas absorbant 3 ne s'étendant pas sur toute la longueur de la couche, comme on peut le voir sur la figure 1.

Bien entendu, l'invention n'est pas limitée à l'exemple de réalisation qui vient d'être décrit.

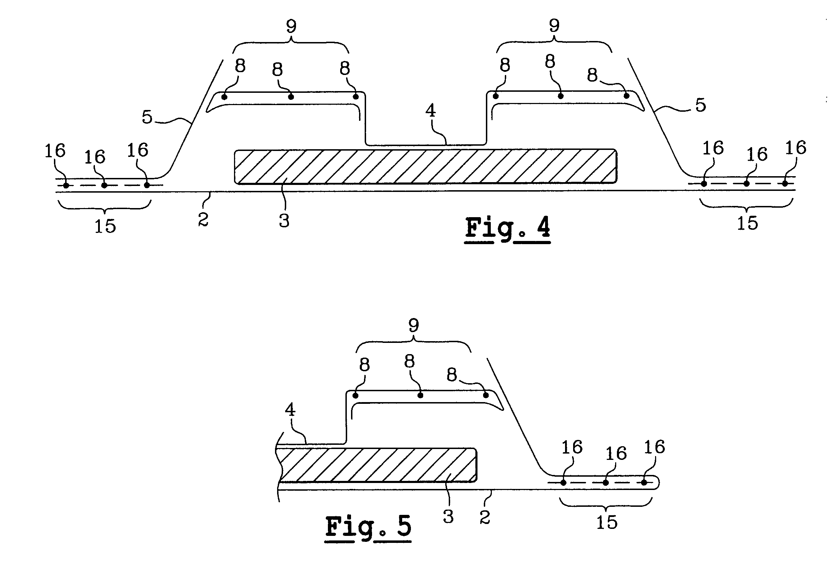

On peut notamment, comme illustré sur la figure 4, disposer des élastiques entre les voiles de liaison 5 et la feuille de fond 2, de manière à constituer des bandes de jambe 15 élastiquement susceptibles de contraction.

De telles bandes de jambe réduisent les risques de fuite de liquides.

Dans les exemples qui viennent d'être décrits, les voiles de liaison 5 sont constitués par des éléments rapportés.

On peut encore, dans une variante illustrée à la figure 5, réaliser les voiles de liaison par pliage de la feuille de fond 2.

## Revendications

1. Couche (1) à usage unique, comportant une feuille de fond (2), un matelas absorbant (3) et une feuille supérieure (4) perméable aux liquides, **caractérisée par le fait que** cette couche (1) comporte en outre deux bandes intérieures (9) pourvues d'élastiques sur leur largeur, au moins dans la région d'entrejambe, un bord latéral de chaque bande (9) étant assujetti au matelas absorbant (3) et l'autre bord latéral étant assujetti à un voile de liaison (5) solidaire de la feuille de fond (2), **par le fait que** ladite feuille supérieure (4) comporte des parties latérales (4b), rabattues sur elles-mêmes autour d'élastiques (8) pour constituer les bandes intérieures, et **par le fait que** chaque voile de liaison (5) comporte un bord distal libre (10), chaque bande intérieure (9) étant fixée au voile de liaison (5) correspondant, en retrait de son bord distal et sur sa face intérieure.

2. Couche selon la revendication précédente, **caractérisée par le fait que** chaque voile de liaison (5) est assemblé avec la feuille de fond (2) autour d'au moins un élastique (16) de manière à constituer une bande de jambe.

3. Couche selon l'une des deux revendications précédentes, **caractérisée par le fait que** les bandes intérieures (9) s'étendent au-dessus du matelas absorbant.

4. Couche selon l'une quelconque des revendications précédentes, **caractérisée par le fait que** les bandes intérieures (9) sont réalisées d'un seul tenant avec la feuille supérieure.

5. Couche selon la revendication précédente, **caractérisée par le fait que** la feuille supérieure (4) est fixée au matelas absorbant le long d'une région centrale (4a) de ce dernier s'étendant entre les bandes intérieures (9).

6. Couche selon l'une quelconque des revendications précédentes, **caractérisée par le fait que** la feuille supérieure (4) est hydrophile.

7. Couche selon l'une quelconque des revendications précédentes, **caractérisée par le fait que** la feuille de fond (2) est imperméable.

8. Couche selon l'une quelconque des revendications précédentes, **caractérisée par le fait que** les voiles de liaison (5) sont constitués par un non-tissé hydrophobe.

9. Couche selon l'une quelconque des revendications précédentes, **caractérisée par le fait que** les voiles de liaison (5) sont des éléments rapportés.

10. Couche selon l'une quelconque des revendications précédentes, **caractérisée par le fait que** les voiles de liaison (5) sont réalisés dans le même matériau que la feuille de fond (2).

11. Couche selon la revendication précédente, **caractérisée par le fait que** les voiles de liaison (5) sont formés après pliage de la feuille de fond (2).
